# EUROPÄISCHE PATENTSCHRIFT

(11) **EP 0 698 117 B1**
(45) Veröffentlichungstag und Bekanntmachung des Hinweises auf die Patenterteilung: **04.12.2002**
(21) Anmeldenummer: 94916161.6
(22) Anmeldetag: 28.04.1994
(51) Int. Cl.: C12P 21/08, C12N 5/00, G01N 33/573

(54) **MONOKLONALE ANTIKÖRPER GEGEN CK-MB**
MONOCLONAL ANTIBODIES AGAINST CK-MB
ANTICORPS MONOCLONAUX CONTRE L'ISOENZYME CK-MB

(30) Priorität: 30.04.1993 DE 4314254
(43) Veröffentlichungstag der Anmeldung: 28.02.1996
(60) Teilanmeldung: 02012972.2
(73) Patentinhaber: Roche Diagnostics GmbH, 68298 Mannheim (DE)
(72) Erfinder: HÜBNER-PARAJSZ, Christa, D-82327 Tutzing (DE); ESSIG, Ulrich, D-82152 Planegg (DE); LANG, Fridl, D-82327 Tutzing (DE); VOGEL, Rudolf, D-82362 Weilheim (DE)
(86) Internationale Anmeldenummer: EP9401337
(87) Internationale Veröffentlichungsnummer: WO94025617

(56) Entgegenhaltungen:
- EP-A- 0 228 174
- AMERICAN HEART JOURNAL, Bd.122, Nr.4, Oktober 1991 Seiten 958 - 964 D. WOLFSON ET AL. 'Three rapid immunoassays for the detemination of creatine kinase MB'
- CLIN. CHEM., Bd.36, Nr.9, 1990 Seiten 1679 - 1683 E. S. LIANIDOU ET AL. 'Assay of creatine kinase isoenzyme MB in serum with time-resolved immunofluorometry'
- CLIN. CHEM., Bd.33, Nr.9, 1987 Seiten 1517 - 1520 U. PIRAN ET AL. 'Immunochemiluminometric Assay of creatine kinase MB with a monoclonal antibody to the MB isoenzyme'
- ANN. CLIN. BIOCHEM., Bd.29, 1992 Seiten 563 - 564 M. N. BADMINGTON ET AL. 'Evaluation of a new rapid immunometric method for the measurement of the MB isoenzyme of creatine kinase in serum'
- CLIN. CHEM., Bd.32, Nr.4, 1986 Seiten 657 - 663 H. C. VAIDYA ET AL. 'Direct measurement of creatine kinase MB activity in serum after extraction with a monoclonal antibody specific to the MB isoenzyme'
- APPLE F, ET AL.: 'Clinical and analytical evaluation of two immunoassays for direct measurement of creatine kinase MB with two monoclonal anti-CK-MB antibodies.' CLINICAL CHEMISTRY Bd. 34, Nr. 11, 1988, Seiten 2364 - 2367

## Beschreibung

Die Erfindung betrifft monoklonale Antikörper, die an das CK-MB Isoenzym, nicht jedoch an die B- oder M-Untereinheit der CX-MB oder an die CK-MM und CK-BB Isoenzyme binden, sowie ein Verfahren zum Nachweis von CK-MB in einem diagnostischen Test unter Verwendung dieser Antikörper.

Die Creatinkinase (ATP: Creatin-N-phosphotransferrase, EC 2.7.3.2) katalysiert die reversible Phosphorylierung von Creatin in Gegenwart von ATP zu Creatinphosphat. Das Enzym besteht aus zwei Untereinheiten, von denen eine M-(muskelspezifische) und B-(gehirnspezifische) Form bekannt ist, die untereinander eine Homologie von 78,7 % aufweisen. Das dimere Enzym besteht entweder aus zwei identischen Untereinheiten (muskelspezifisches CK-MM Isoenzym und gehirnspezifisches CK-BB Isoenzym) oder liegt als Heterodimer vor. Diese heterodimere Isoenzym CK-MB ist spezifisch für den Herzmuskel und vor allem bei einer Herzmuskelschädigung wie z.B. einem Herzinfarkt, einer progressiven Muskeldystrophie oder toxisch bedingten Myopathien im Serum nachweisbar. Aufgrund der hohen Spezifität ist die Bestimmung von CK-MB im Serum ein wichtiger Notfallparameter für die Diagnose eines Herzinfarkts. Es sind daher bereits einige Verfahren zur Bestimmung von CK-MB im Serum beschrieben worden, bei denen CK-MB über eine elektrophoretische oder ionenchromatographische Auftrennung, über einen Radioimmunoassay oder eine enzymatische Aktivitätsbestimmung unter Hemmung der M-Untereinheit mit einem Antikörper nachgewiesen wird. Diese Verfahren sind jedoch sehr zeitaufwendig. Da die Bestimmung der CK-MB insbesondere in Notfallsituationen von Bedeutung ist, muß ein geeigneter Test nicht nur spezifisch für CK-MB, sondern auch schnell durchführbar sein.

Es wurden daher auch Immunoassays beschrieben, bei welchem CK-MB über einen Sandwich-Assay mit Antikörpern gegen CK-MM und CK-BB nachgewiesen wird. Obwohl hierdurch theoretisch nur CK-MB nachweisbar sein sollte, hat sich ein derartiger Sandwich-Assay als sehr störanfällig gegenüber CK-MM und CK-BB erwiesen, da Anti-CK-MM-Antikörper auch mit CK-BB und Anti-CK-BB-Antikörper auch mit CK-MM kreuzreagieren. Daher führt ein solcher Sandwich-Assay leicht zu falsch-positiven Ergebnissen. Von Vaidya et al. (Clin. Chem. 32, 1986, 657 - 663) wurden auch für CK-MB spezifische monoklonale Antikörper entwickelt. Von diesen Antikörpern wurde einer besonders gut charakterisiert. Dieser Antikörper wird als sogenannter "Conan"-Antikörper bezeichnet und gilt als Referenzantikörper für monoklonale Antikörper gegen CK-MB. Dieser sowie eine Reihe weiterer inzwischen entwickelter monoklonaler Antikörper gegen CK-MB weisen jedoch nur eine geringe Affinität zu CK-MB auf und erkennen zudem alle das gleiche Epitop, das sogenannte "Conan-"Epitop von CK-MB, so daß mit diesen Antikörpern kein Sandwich-Immunoassay möglich ist.

Bei den bislang beschriebenen Sandwich-Immunoassays wird nur ein CK-MB-spezifischer monoklonaler Antikörper verwendet. Der zweite Antikörper ist ein Antikörper gegen die M-oder B-Untereinheit und erkennt somit auch CK-MM bzw. CK-BB.

Aufgabe der Erfindung war es daher, monoklonale Antikörper gegen das CK-MB Isoenzym zur Verfügung zu stellen, die nicht an die CK-MM und CK-BB Isoenzyme binden.

Diese Aufgabe wird gelöst durch einen monoklonalen Antikörper, der an das CK-MB Isoenzym, nicht jedoch an die B- oder M-Untereinheit allein der CK-MB oder wesentlich an die CK-MM und CK-BB Isoenzyme bindet, wobei die Bindung an CK-MB in äquivalenter Weise erfolgt wie die Bindung der monoklonalen Antikörper DSM ACC 2058, DSM ACC 2060 oder DSM ACC 2168. Dieser monokolnale Antikörper ist erhältlich durch Immunisierung von A/J-Mäusen (H-2^{a} Haplo-typ) mit dem CK-MB Isoenzym unter Verwendung von Aluminium-hydroxid als Adjuvans, Immortalisierung der Milzzellen der immunisierten Mäuse und Klonierung derjenigen immortali-sierten Zellen, die einen Antikörper produzieren, der an CK-MB, nicht aber an CK-MM oder CK-BB bindet und vom "Conan-MB-Antikörper" (ATCC HB 8939) nicht von der Bindung an CK-MB verdrängt wird.

Ein solcher Antikörper, der spezifisch mit CK-MB, aber nicht wesentlich mit CK-MM oder CK-BB reagiert, muß ein Konformationsepitop erkennen, das sowohl von der M-, als auch von der B-Untereinheit gebildet wird. Da die Bindungsregion eines Antikörpers etwa 6 bis 10 Aminosäuren des Antigens als Epitop erkennt, tragen die beiden Untereinheiten des CK-MB zu diesem Konformationsepitop nur jeweils etwa 5 Aminosäuren bei. Zudem sind aufgrund der sterischen Anordnung der Aminosäureseitenketten sowie deren Hydrophilie nur bestimmte Abschnitte einer Proteinsequenz immunogen wirksam. Daher weisen dimere Antigene in der Regel nur eine sehr geringe Anzahl an Konformationsepitopen auf, zu deren Spezifität die beiden Untereinheiten gemeinsam beitragen. Es war daher überraschend, daß monoklonale Antikörper gegen das CK-MB Isoenzym gefunden werden konnten, welche vier verschiedene Konformationsepitope auf dem CK-MB Isoenzym erkennen und somit für einen Sandwich-Assay oder sogar eine Tripletbildung geeignet sind. Diese vier Epitope werden charakterisiert durch die Bindung des "Conan-MB-Antikörpers" ATCC HB 8939 sowie der erfindungsgemäßen monoklonalen Antikörper DSM ACC 2059 und/oder DSM ACC 2057 für das erste Epitop, durch die Bindung des Antikörpers DSM ACC 2058 für das zweite Epitop, durch die Bindung des Antikörpers DSM ACC 2060 für das dritte Epitop und durch die Bindung des Antikörpers DSM ACC 2168 für das vierte Eptiop.

Es hat sich überraschenderweise gezeigt, daß die Antikörper DSM ACC 2058, DSM ACC 2060, DSM ACC 2057, DSM ACC 2059 und DSM ACC 2168 eine höhere Assoziationskonstante für die Bindung an CK-MB aufweisen als die bislang bekannten monoklonalen Antikörper gegen CK-MB. Diese monoklonalen Antikörper reagieren somit sehr schnell mit CK-MB und sind daher für Schnelldiagnostiktests besonders geeignet.

Bevorzugt ist daher ein erfindungsgemäßer monoklonaler Antikörper gegen das CK-MB Isoenzym, welcher eine Assoziationskonstante für die Bindung an CK-MB von mindestens 1 x 10⁵ mol⁻¹ x 1 x sec⁻¹ aufweist.

Vorzugsweise sind die erfindungsgemäßen monoklonalen Antikörper vom IgGl-Isotyp.

Die erfindungsgemäßen monoklonalen Antikörper gegen CK-MB können in beliebiger Weise sowohl miteinander als auch mit bekannten Antikörpern gegen CK-MB für einen Sandwich-Immunoassay zum Nachweis von CK-MB kombiniert werden, sofern zwei Antikörper verwendet werden, welche ein unterschiedliches Epitop auf CK-MB erkennen. Die erfindungsgemäßen Antikörper können natürlich auch mit Antikörpern gegen die M- bzw. B-Untereinheit der Kreatinkinase kombiniert werden.

Für einen möglichst sensitiven Test sollten die Antikörper zudem eine hohe Affinität zu CK-MB aufweisen. Es hat sich nun überraschenderweise gezeigt, daß die oben genannten monoklonalen Antikörper DSM ACC 2059 und DSM ACC 2057 eine höhere Affinitätskonstante aufweisen als die bislang bekannten Antikörper gegen dieses Epitop von CK-MB.

Ein weiterer Gegenstand der Erfindung ist daher ein monoklonaler Antikörper, der an das CK-MB Isoenzym, nicht aber an die B- oder M-Untereinheit allein der CK-MB oder nicht wesentlich an die CK-MM und CK-BB Isoenzyme bindet, wobei die Bindung an CK-MB in äquivalenter Weise erfolgt wie die Bindung der monoklonalen Antikörper DSM ACC 2057 oder DSM ACC 2059, der eine Affinitätskonstante von mindestens 1 x 10⁸ mol⁻¹ x 1 zu CK-MB aufweist. Dieser monoklonale Antikörper ist erhältlich durch Immunisierung von A/J-Mäusen (H-2^{a} Haplotyp) mit dem CK-MB Isoenzym unter Verwendung von Aluminiumhydroxid als Adjuvans, Immortalisierung der Milzzellen der immunisierten Mäuse und Klonierung derjenigen immortalisierten Zellen, die einen Antikörper produzieren, der an CK-MB, nicht aber wesentlich an CK-MM oder CK-BB bindet, vom "Conan-MB-Antikörper" (ATCC HB 8939) von der Bindung an CK-MB verdrängt wird und eine Affinitätskonstante von mindestens 1 x 10⁸ mol⁻¹ x 1 zu CK-MB aufweist. Die Bestimmung der Affinitätskonstante erfolgt dabei über das BIAcore® -System (Pharmacia LKB) unter Verwendung der Kinetic Evaluation Kit Software (Pharmacia LKB, Ident.-Nr. BR-1000-19).

Die oben genannten monoklonalen Antikörper DSM ACC 2057 und DSM ACC 2059 weisen zudem eine Dissoziationskonstante von weniger als 4 x 10⁻⁴ sec⁻¹ auf. Dies bedeutet, daß der Komplex aus Antikörper und CK-MB nur sehr langsam wieder dissoziiert. Dies ist für bestimmte immunologische Testverfahren wie z.B. das IEMA (Immuno Enzyme Metric Assay) wichtig, da eine schnelle Dissoziation des Antikörper-CK-MB-Komplexes zu einer Konkurrenzreaktion zwischen der Bindung des markierten Antikörpers an den Analyten bzw. das immobilisierte Antigen und damit zu einer Verfälschung des Meßwertes führen würde.

Ein bevorzugter Gegenstand der Erfindung ist daher ein erfindungsgemäßer monoklonaler Antikörper gegen das CK-MB Isoenzym, welcher in äquivalenter Weise an CK-MB bindet wie der monoklonale Antikörper DSM ACC 2057 oder DSM ACC 2059 und der eine Dissoziationskonstante von weniger als 4 x 10⁻⁴ sec⁻¹ aufweist. Besonders bevorzugt sind solche erfindungsgemäßen monoklonalen Antikörper, welche zudem eine Assoziationskonstante für die Bindung an CK-MB von mindestens 1 x 10⁵ mol⁻¹ x 1 x sec⁻¹ aufweisen.

Die erfindungsgemäßen Antikörper können erhalten werden durch Immunisierung von A/J-Mäusen (H-2^{a} Haplotyp) mit dem CK-MB-Isoenzym. Es hat sich überraschenderweise herausgestellt, daß bei Immunisierung dieses Mausstammes ein wesentlich höherer Antikörpertiter erhalten wird als mit den üblicherweise hierzu verwendeten Balb/c-Mäusen. Weiterhin hat es sich als besonders vorteilhaft erwiesen, zur Herstellung der erfindungsgemäßen Antikörper gegen ein Konformationsepitop das CK-MB zur Immunisierung an Aluminiumhydroxid und Bordetella pertussis zu adsorbieren. Nach etwa vier Monaten werden die Milzzellen der immunisierten Mäuse immortalisiert und diejenigen immortalisierten Zellen kloniert, die den gewünschten Antikörper produzieren. Die Immortalisierung der Milzzellen erfolgt nach dem Fachmann bekannten Verfahren, vorzugsweise werden die Zellen mit Myelomzellen fusioniert. Diejenigen immortalisierten Zellen, die den gewünschten Antikörper produzieren, werden durch parallele Bestimmung der Reaktivität einer Probe des Kulturüberstands mit immobilisiertem CK-MB, CK-MM bzw. CK-BB identifiziert. Die gewünschten Antikörper gegen ein Konformationsepitop von CK-MB reagieren mit CK-MB, aber nicht wesentlich mit CK-MM oder CK-BB. Die Bezeichnung nicht wesentlich bedeutet, daß die Reaktion mit CK-MM und CK-BB derart niedrig liegt, daß in einem immunologischen Test zum Nachweis von CK-MB die durch das Vorhandensein der CK-MM und CK-BB in deren physiologisch vorkommenden Konzentration hervorgerufene Störungen weniger als 1 %, bevorzugt weniger als 0,1 % betragen. Die Reaktion mit CK-MM muß dabei verschwindend gering sein, da CK-MM im Serum oder Plasma in hohen Konzentrationen vorkommt. Da CK-BB im Serum oder Plasma nur in sehr geringen Konzentrationen vorkommt, ist eine geringe Reaktion der Antikörper mit diesem Isoenzym nicht relevant. Die entsprechenden immortalisierten Zellen werden nach üblichen Verfahren, z.B. durch Vereinzelung über einen fluoreszenzaktivierten Zellsorter kloniert. Zur Identifizierung derjenigen Klone, die einen Antikörper produzieren, welcher in äquivalenter Weise an CK-MB bindet wie der monoklonale Antikörper DSM ACC 2058, DSM ACC 2060 oder DSM ACC 2168, wird ein kompetitives Testsystem verwendet. Dazu wird zum Beispiel mit Hilfe eines Enzym-Immunoassays überprüft, inwieweit der Antikörper mit dem "Conan-MB-Antikörper" (ATCC HB 8939) um die Bindung an CK-MB konkurriert. Dazu inkubiert man CK-MB mit dem "Conan-MB-Antikörper" (ATCC HB 8939) in markierter Form und einem Überschuß des in Betracht gezogenen Antikörpers. Durch Immobilisierung der gebildeten Komplexe, Trennung der festen von der flüssigen Phase und Nachweis der gebundenen Markierung in einer der beiden Phasen kann dann leicht festgestellt werden, ob der in Betracht gezogene Antikörper vom "Conan-MB-Antikörper" (ATCC HB 8939) aus der Bindung verdrängt wird. Ist dies nicht der Fall, so liegt eine Bindung an CK-MB vor, die mit hoher Wahrscheinlichkeit der Bindung des monoklonalen Antikörpers DSM ACC 2058, DSM ACC 2060 oder DSM ACC 2168 in äquivalenter Weise entspricht.

Zur Identifizierung von solchen Klonen, die einen Antikörper der gewünschten Assoziationskonstante oder Dissoziationskonstante der Bindung an CK-MB bzw. der gewünschten Affinität zu CK-MB produzieren, wird dann in einer Probe des Kulturüberstands die Assoziationskonstante, Dissoziationskonstante bzw. Affinität des gebildeten Antikörpers zu CK-MB bestimmt. Diese Bestimmung erfolgt vorzugsweise über das BIAcore 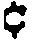 -System (Pharmacia LKB, Kinetic Evaluation Kit, Ident.-Nr. BR-1000-19). Auf diese Weise konnten die Hybridomzellinien DSM ACC 2059, DSM ACC 2057, DSM ACC 2058, DSM ACC 2060 und DSM ACC 2168 erhalten werden.

Ein besonders bevorzugter Gegenstand der Erfindung sind monoklonale Antikörper, die erhältlich sind aus einer der Hybridomlinien DSM ACC 2059, DSM ACC 2057, DSM ACC 2058, DSM ACC 2060 und/oder DSM ACC 2168 sowie die genannten Hybridomlinien.

Ein weiterer Gegenstand der Erfindung ist ein Verfahren zur Herstellung monoklonaler Antikörper, die an das CK-MB Isoenzym, nicht aber an die B- oder M-Untereinheit allein der CK-MB oder nicht wesentlich an die CK-MM- und CK-BB-Isoenzyme binden, wobei diese Bindung an CK-MB in äquivalenter Weise erfolgt wie die Bindung der monoklonalen Antikörper DSM ACC 2058, DSM ACC 2060 oder DSM ACC 2168, durch Immunisierung von A/J-Mäusen (H-2^{a} Haplotyp) mit dem CK-MB-Isoenzym unter Verwendung von Aluminiumhydroxid als Adjuvans, Immortalisierung der Milzzellen der immunisierten Mäuse, Klonierung derjenigen immortalisierten Zellen, die einen Antikörper produzieren, der an CK-MB, aber nicht wesentlich an CK-MM oder CK-BB bindet und vom "Conan-MB-Antikörper" (ATCC HB 8939) nicht von der Bindung an CK-MB verdrängt wird, und Isolierung des monoklonalen Antikörpers aus den klonierten Zellen oder deren Kulturüberstand nach bekannten Verfahren.

In einer besonderen Ausführungsform des erfindungsgemäßen Verfahrens werden solche Klone ausgewählt, die einen Antikörper produzieren, der eine Assoziationskonstante für die Bindung an CK-MB von mindestens 1 x 10⁵ mol⁻¹ x 1 x sec⁻¹ oder eine Dissoziationskonstante von weniger als 4 x 10⁻⁴ sec⁻¹ oder eine Affinitätskonstante von mindestens 1 x 10⁸ mol⁻¹ x 1 aufweist.

Mit Hilfe der erfindungsgemäßen monoklonalen Antikörper sind nun auch solche immunologischen Testverfahren zur diagnostischen Bestimmung von CK-MB möglich, die zwei für CK-MB spezifische Antikörper hoher Affinität gegen verschiedene Epitope erfordern.

Ein weiterer Gegenstand der Erfindung ist daher die Verwendung eines erfindungsgemäßen monoklonalen Antikörpers zur Bestimmung von CK-MB in einem diagnostischen Test.

Diese Bestimmung kann über alle gängigen Immunoassays wie z.B. ELISA, Fluoreszenz-Immunoassay, Elektrochemilumineszenz-Immunoassay (WO 86/02734, WO 87/06706, WO 92/14138), Radioimmunoassay, Fluoreszenz-Polarisations-Immunoassay, CEDIA (Henderson et al., Clin. Chem. 32 (1986), 1637 - 1641 und US 4,708,929), IEMA oder EMIT erfolgen. Der Test kann dabei sowohl als homogener Test, z.B. über einen kompetitiven Immunoassay, als auch als heterogener Test durchgeführt werden. Vorzugsweise erfolgt der Test unter Immobilisierung einer der Reaktionspartner. Besonders bevorzugt ist eine Bestimmung über den sogenannten LPIA-Test (Latex Particle Immunoassay). Dazu werden zwei Antikörper gegen verschiedene Epitope des CK-MB zunächst an eine universelle Streptavidin-Latex-Matrix gebunden. Die Bestimmung von CK-MB erfolgt dann durch Inkubation dieser Konjugate mit der Probe bzw. einem CK-MB-Standard und Messung der durch die Agglutination bewirkten Extinktionszunahme.

Ein weiterer Gegenstand der Erfindung ist daher ein Verfahren zum diagnostischen Nachweis von CK-MB in einem homogenen diagnostischen Test, in dem mindestens ein erfindungsgemäßer Antikörper an einen Latexpartikel gebunden wird und nach Inkubation mit der Probelösung sowie einem weiteren latexgebundenen Antikörper, der an CK-MB bindet, ohne den ersten Antikörper aus seiner Bindung an CK-MB zu verdrängen, die Agglutination der latexgebundenen Antikörper als Maß für die Konzentration von CK-MB in der Probe bestimmt wird.

Als Probelösung wird hierbei üblicherweise eine Serumprobe verwendet. Als zweiter Latexpartikel-gebundener Antikörper kann prinzipiell jeder Antikörper verwendet werden, welcher mit CK-MB reagiert, also auch ein Antikörper gegen die B-oder M-Untereinheit der Kreatinkinase. Vorzugsweise wird jedoch auch als zweiter latexgebundener Antikörper ein erfindungsgemäßer hochaffiner Antikörper gegen ein Konformationsepitop von CK-MB verwendet. Besonders bevorzugt wird als einer der beiden latexgebundenen Antikörper ein Antikörper verwendet, welcher von der Hybridomzellinie DSM ACC 2058, DSM ACC 2060 oder DSM ACC 2168 produziert wird. Die Antikörper können dabei sowohl als vollständige Antikörper sowie als funktionelle Antikörperfragmente eingesetzt werden. Vorzugsweise werden Fab'-Fragmente eingesetzt.

Ein weiterer Gegenstand der Erfinding ist ein Verfahren zum Nachweis von CK-MB in einem homogenen diagnostischen Test nach dem CEDIA-Verfahren, in dem ein erfindungsgemäßer Antikörper eingesetzt wird. Beim CEDIA-Verfahren werden zum Nachweis bestimmte Enzyme wie β-Galaktosidase verwendet, die in zwei jeweils enzymatisch inaktive Bestandteile, ein großes Polypeptid (Enzymakzeptor EA) und ein kleines Polypeptid (Enzymdonor ED), vorliegen. Diese Bestandteile assoziiren spontan zu dem enzymatisch aktiven Protein. An ED wird ein Peptidantigen, im Falle von CK-MB, dieses Isoenzym oder ein Fragment davon, das die CK-MB spezifischen Epitope enthält, derart gebunden, daß die Assoziation von ED mit EA nicht verhindert wird. Diese Assoziation wird dann gehemmt, wenn an den Peptidantigen-ED-Komplex ein Antikörper gegen das Peptidantigen bindet. In einer Reagenzlösung, in der EA, Peptidantigen-ED-Komplex und ein Peptidantigen-spezifischer Antikörper vorliegen, kann kein aktives Enzym gebildet werden. Nach Zugabe der Probelösung, die Peptidantigene beziehungsweise beim CK-MB-Nachweis das CK-MB koenzym enthält, konkurriert CK-MB mit den Peptidantigen-ED-Komplex um die Bindung an den erfindungsgemäßen Antikörper, wodurch sich der aktive ED/EA-Komplex ausbilden kann. Das Meßsignal ist somit proportional der Menge des in der Probe vorhandenen CK-MB-Isoenzyms. Bei dem CEDIA-Verfahren ist ein Antikörper, der an CK-MB in äquivalenter Weise bindet wie die monoklonalen Antikörper DSM ACC 2057 oder DSM ACC 2059 und eine Affinitätskonstante von mindestens 1 x 10⁸ mol⁻¹ x 1 zu CK-MB aufweisen, gut geeignet. Besonders bevorzugt ist der monoklonale Antikörper DSM ACC 2059, der eine besonders hohe Affinitätskonstante von 7,0 x 10⁸ mol⁻¹ x 1 aufweist.

Ein weiterer Gegenstand der Erfindung ist ein Verfahren zum diagnostischen Nachweis von CK-MB über einen Sandwichassay, in dem mindestens ein erfindungsgemäßer Antikörper an eine feste Phase gebunden wird, mit der Probelösung sowie einem weiteren markierten Antikörper, der an CK-MB bindet, ohne den ersten Antikörper aus seiner Bindung an CK-MB zu verdrängen, inkubiert wird und nach Trennung von fester und flüssiger Phase die Markierung in einer der beiden Phasen als Maß für die Konzentration von CK-MB in der Probe bestimmt wird. Vorzugsweise wird dabei als einer der beiden Antikörper ein Antikörper verwendet, welcher von der Hybridomlinie DSM ACC 2058, DSM ACC 2060 oder DSM ACC 2168 produziert wird.

Die Immobilisierung des ersten Antikörpers an die feste Phase erfolgt in der dem Fachmann bekannten Weise. Vorzugsweise wird der Antikörper biotinyliert und an eine mit Streptavidin beschichtete feste Phase gebunden. Die Markierung des zweiten Antikörpers erfolgt in üblicher Weise, z.B. über direkte Markierung mit einem Enzym, einem Fluoreszenz- oder Chemilumineszenzfarbstoff oder durch Bindung eines weiteren Antikörpers, welcher gegen den zweiten Antikörper gerichtet ist und der in entsprechender Weise markiert wurde.

Bevorzugt ist weiterhin der Nachweis über den Elektrochemilumineszenz(ECL)-Immunoassay, wie in WO 85/02734, WO 87/06706 oder WO 92/14138 beschrieben. Dazu wird ein Antikörper gegen CK-MB zunächst an ein Magnetpartikel gebunden Bevorzugt erfolgt die Bindung über Streptavidin und Biotin. Es werden Streptavidinbeschichtete Magnetpartikel und ein biotinylierter CK-MB-Antikörper eingesetzt. Der zweite erfindungsgemäße Antikörper gegen CK-MB soll ein anderes Epitop als der erste Antikörper erkennen. Dieser zweite Antikörper wird mit dem Label gekuppelt. Als Label wird eine elektrochemilumineszierende Verbindung, wie in der WO 86/2734 und WO 87/06706 beschrieben, verwendet. Bevorzugt wird als Label Tris-(Bispyridyl)Ruthenium verwendet. Die Kopplung des Labels an den Antikörper erfolgt nach Methoden des Standes der Technik. Die Inkubation der beiden erfindungsgemäßen Antikörper mit der Probe und den Magnetpartikeln kann gleichzeitig oder nacheinander erfolgen. Zur Messung wird ein Apparat, wie in der WO 90/11511 beschrieben, benutzt.

Bevorzugt wird bei dem ECL-Immunoassay ein erster Antikörper verwendet, der an CK-MB in äquivalenter Weise bindet, wie die monoklonalen Antikörper DSM ACC 2059 oder DSM ACC 2057. Der zweite Antikörper bindet an ein anderes Epitop und bevorzugt in äquivalenter Weise wie der monoklonale Antikörper DSM ACC 2058.

Bei dem Sandwich-Immunoassay hat sich die Verwendung zweier monoklonaler Antikörper, die beide an das CK-MB Isoenyzm binden, nicht aber an die B- oder M-Untereinheit allein des CK-MB Isoenzyms, und nicht wesentlich an die CK-MM und CK-BB Isoenyzme binden und die nicht miteinander um die selbe Bindestelle an CK-MB konkurrieren und von denen einer mindestens eine Affinitätskonstante von mindestens 1 x 10⁸ mol⁻¹ x 1 zu CK-MB aufweist, als besonders vorteilhaft erwiesen. Besonders bevorzugt weist mindestens einer dieser Antikörper eine Assoziationskonstante von mindestens 1,9 x 10⁵ mol⁻¹ x 1 x sec⁻¹ auf.

Die oben genannten erfindungsgemäßen Hybridomzellinien DSM ACC 2059, DSM ACC 2057, DSM ACC 2058 und DSM ACC 2060 wurden am 03.02.1993 und die Hybridomzellinie DSM ACC 2168 am 19.04.1994 bei der Deutschen Sammlung für Zellkulturen und Mikroorganismen GmbH, Mascheroder Weg 1 b,
D-38124 Braunschweig hinterlegt.

Die Erfindung wird durch die folgenden Beispiele näher erläutert.

### Beispiel 1

### Herstellung monoklonaler Antikörper gegen das CK-MB-Isoenzym

### a) Immunisierung von Mäusen

12 Wochen alte A/J-Mäuse werden mit 100 µg CK-MB (Fa. Aalto, Dublin) intraperitoneal erstimmunisiert. Nach 6 Wochen folgen zwei weitere Immunisierungen intraperitoneal in monatlichen Abständen. Dabei wird jeder Maus 100 µg CK-MB an Aluminiumhydroxid (Firma Paesel, Frankfurt) adsorbiert sowie 10⁹ Keime Bordetella pertussis (Fa. Behring, Frankfurt) verabreicht. Anschließend erfolgen die letzten beiden Immunisierungen intravenös mit je 100 µg CK-MB in PBS-Puffer am 3. und 2. Tag vor der Fusion.

### b) Fusion und Klonierung

Die Fusion von Milzzellen der gemäß 1. immunisierten Mäuse mit Myelomzellen erfolgt in Anlehnung an Galfré, Methods in Enzymology 73, 1981, 3. Dabei werden ca. 1*10⁸ Milzzellen der immunisierten Maus mit 2*10⁷ Myelomzellen (P3X63-Ag8-653, ATCC CRL1580) gemischt und abzentrifugiert (10 min bei 300 g und 4°C). Die Zellen werden dann einmal mit RPMI-1640-Medium ohne fötales Kälberserum (FKS) gewaschen und bei 400 g in einem 50 ml Spitzröhrchen erneut abzentrifugiert. Der Überstand wird abgekippt, das Zellsediment durch Klopfen leicht aufgelockert, 1 ml PEG (Molekulargewicht 4000, Merck, Darmstadt) dazugegeben, und durch Pipettieren gemischt. Nach 1 min im Wasserbad bei 37°C werden bei Raumtemperatur 5 ml RPMI 1640 ohne FKS in einem Zeitraum von 4 - 5 min zugetropft. Danach werden 5 ml RPMI 1640 mit 10% FKS in ca. 1 min zugetropft, durchmischt auf 50 ml mit Medium (RPMI 1640 + 10% FKS) aufgefüllt und anschließend 10 min bei 400 g und 4°C zentrifugiert. Die sedimentierten Zellen werden in RPMI 1640 Medium mit 10% FKS aufgenommen und in Hypoxanthin-Azaserin Selektionsmedium (100 mmol/l Hypoxanthin, 1 µg/ml Azaserin in RPMI 1640 + 10 % FKS) eingesät. Als Wachstumsfaktor wird dem Medium Interleukin 6 (Boehringer Mannheim GmbH, Kat.-Nr. 1271 172, 100 U/ml) zugegeben.

Nach ca. 10 Tagen werden die Primärkulturen auf spezifische Antikörpersynthese und Kreuzreaktion getestet (siehe Beispiel 2). CK-MB-positive Primärkulturen, die keine wesentliche Kreuzreaktion mit den Isoenzymen CK-MM und CK-BB zeigen, werden mittels fluoreszenzaktiviertem Zellsorter in 96er-Zellkulturplatten kloniert. Hierbei wird dem Medium als Wachstumszusatz Interleukin-6 (Boehringer Mannheim GmbH, Kat.-Nr. 1271172, 100 U/l) zugegeben. Von denjenigen Klonen, die einen Antikörper gegen ein Konformationsepitop von CK-MB produzieren (Bestimmung gemäß Beispiel 2), werden diejenigen Kulturen ausgewählt, die einen Antikörper mit einer Affinität von mindestens 9 x 10⁷ mol⁻¹ x 1 zu CK-MB aufweisen (Bestimmung gemäß Beispiel 3) und/oder einen Antikörper, der ein anderes Epitop erkennt als der "Conan-MB-Antikörper" (Bestimmung gemäß Beispiel 4).

### c) Immunglobulingewinnung aus den Zellkulturüberständen

Die erhaltenen Hybridomzellen werden mit einer Dichte von 1 x 10⁵ Zellen pro ml in RPMI 1640-Medium mit 10 % FKS eingesät und 7 Tage lang in einem Fermenter (Fa. Thermodux, Wertheim/Main, Modell MCS-104XL, Best.-Nr. 144-050) vermehrt. In dem Kulturüberstand werden Konzentrationen von durchschnittlich 100 µg monoklonaler Antikörper je ml erreicht. Die Reinigung dieses Antikörpers aus dem Kulturüberstand erfolgt nach proteinchemisch üblichen Methoden (z.B. gemäß Methods in Enzymology 121 (1986), 587 - 695).

### Beispiel 2

### Bestimmung der Spezifität der produzierten Antikörper

Um die Spezifität der Antikörper im Kulturüberstand der Hybridomzellen zu bestimmen, wird in drei parallelen ELISA-Ansätzen die Reaktivität mit CK-MB, CK-MM und CK-BB bestimmt. Dazu werden zunächst 96er Mikrotiter-Platten (Nunc) mit 200 µl/well Thermo-RSA-Streptavidin (10 µg/ml Beschichtungspuffer = 0,2 mol/l Natriumcarbonat/Bicarbonat, pH 9,3 - 9,5, Boehringer Mannheim GmbH, Kat.-Nr. 0725559) beschichtet (1 h Inkubation bei Raumtemperatur unter Schütteln) und 1 x mit 0,9 % NaCl/0,05 % Tween 20 gewaschen. Parallel wird CK-MB, CK-MM und CK-BB (Fa. Aalto, Dublin, Kat.-Nr. 10901, 10217 bzw. 10803) mit D-Biotinyl-∈-amidocapronsäure-N-hydroxysuccinimidester (Boehringer Mannheim GmbH, Kat.-Nr. 01008960) gemäß Angaben des Herstellers biotinyliert. Die biotinylierten Creatin-Kinase-Isoenzyme werden in einer Konzentration von 250 ng/ml PBS mit 0,05 % Tween 20/1 % Rinderserumalbumin aufgenommen, jeweils 100 µl/well der mit Thermo-RSA-Streptavidin beschichteten Mikrotiterplatten gegeben und 1 h bei Raumtemperatur unter Schütteln inkubiert. Anschließend wird 3 x mit 0,9 % Natriumchlorid/0,05 % Tween 20 gewaschen. Jeweils 100 µl der zu untersuchenden Antikörperlösung wird in parallelen Ansätzen in jeweils ein mit CK-MB, CK-MM bzw. CK-BB beschichtetes well gegeben und 1 h bei Raumtemperatur unter Schütteln inkubiert. Nach 3-maligem Waschen mit 0,9 % Natriumchlorid/0,05 % Tween 20 wird zum Nachweis von gebundenem Antikörper aus der Probe jeweils 100 µl eines POD-markierten Fab-Fragments eines polyklonalen Antikörpers aus dem Schaf gegen Maus-Fc-τ (Boehringer Mannheim GmbH, Ident.-Nr. 1047523 entsprechend 100 mU) zugegeben, 1 h bei Raumtemperatur unter Schütteln inkubiert und anschließend 3 x mit 0,9 % Natriumchlorid/0,05 % Tween 20 gewaschen. Schließlich werden jeweils 100 µl/well ABTS® (Boehringer Mannheim GmbH, Kat.-Nr. 1204521 und 1204530) zugegeben und nach 30 min. bei Raumtemperatur die Extinktion bei 450/490 nm in einem MR700 Microplate Reader der Firma Dynatech gemessen.

### Beispiel 3

### Bestimmung der Affinität, Assoziations- und Dissoziationskonstante sowie der Sandwich-Gängigkeit der produzierten Antikörper

Die Bestimmung der Affinität, Assoziations- und Dissoziationskonstanten sowie der Sandwich-Gängigkeit der produzierten Antikörper erfolgt nach dem BIAcoreC-System der Firma Pharmacia LKB. Hierbei wird in der Oberflächenschicht eines sogenannten Sensorchips das CK-MB-Isoenzym kovalent gebunden und damit immobilisiert. Bei Überleitung einer Lösung des zu bestimmenden Antikörpers, wobei der Antikörper durch nicht kovalente Wechselwirkungskräfte an das immobilisierte CK-MB gebunden wird, erhöht sich die Massendichte in der Oberflächenschicht. Die Zunahme der Massendichte läßt sich mittels "Surface Plasmon Resonance" direkt verfolgen. Je nach Anzahl der vorhandenen Bindungsstellen auf dem immobilisierten Reaktionspartner können sich auch noch dritte und vierte Reaktionspartner anlagern. Deren Bindung kann ebenso wie die des ersten Reaktionspartners in Bezug auf Kinetik und Menge beobachtet werden.

Alle Reaktionspartner mit Ausnahme des kovalent gebundenen CK-MB lassen sich ohne Schädigung von CK-MB mit einfachen Mitteln wieder ablösen, so daß an derselben Primärphase weitere Bindungsexperimente unter identischen Randbedingungen durchgeführt werden können.

Zur Bindung von CK-MB an die Oberflächenschicht des Sensorchips wird eine Lösung von 20 µg/ml CK-MB (Alto, Lot No. 20410) in 10 mmol/l HEPES/3,4 mmol/l EDTA/150 mmol/l NaCl/pH 7,4 mit einer Flußrate von 2 µl/min über den Sensorchip geleitet.

Anschließend werden die folgenden Antikörper zugegeben und die Assoziations- bzw. Dissoziationskonstanten für die Bindung an CK-MB sowie die Affinität der Antikörper zu CK-MB über das BIAcore®-System gemäß Angaben des Herstellers bestimmt (Pharmacia LKB, Software Kinetic Evaluation Kit, Ident.-Nr. BR-1000-19). Die so ermittelten Werte der erfindungsgemäßen Antikörper sowie des "Conan-MB-Antikörpers" ATCC HB 8939 und eines kommerziell erhältlichen Antikörpers gegen CK-MB (BP 172-A2720 von Bios Pacific) sind in der folgenden Tabelle 1 zusammengefaßt.

**Tabelle 1**

| Antikörper | Assoziationskonstante (mol⁻¹ xlxsec⁻¹) | Dissoziationskonstante (sec⁻¹) | Affinitätskonstante (mol⁻¹ xl) |
|---|---|---|---|
| DSM ACC 2059 | 1,9 x 10⁵ | 2,7 x 10⁻⁴ | 7,0 x 10⁸ |
| DSM ACC 2057 | 4,4 x 10⁴ | 1,8 x 10⁻⁴ | 2,4 x 10⁸ |
| DSM ACC 2058 | 1,8 x 10⁵ | 3,5 x 10⁻³ | 5,0 x 10⁷ |
| DSM ACC 2060 | 6,4 x 10⁴ | 2,9 x 10⁻³ | 2,2 x 10⁷ |
| ATCC HB 8939 | 3,4 x 10⁴ | 4,1 x 10⁻⁴ | 8,2 x 10⁷ |
| BP173-A2720 (Bios Pacific) | 4,1 x 10⁴ | 1,7 x 10⁻³ | 2,5 x 10⁷ |

In analoger Weise kann über das BIAcore®-System die Sandwich-Gängigkeit der erfindungsgemäßen Antikörper bestimmt werden. Hierzu wird zunächst als Fangantikörper ein polyklonaler Antikörper gegen murines IgGl oder ein polyklonaler Antikörper gegen murines Fcτ am Sensorchip immobilisiert. Anschließend wird zunächst mit einem ersten zu untersuchenden Antikörper gegen CK-MB bis zu einer Menge von 1000 Resonance Units beladen und noch freier Fangantikörper durch Überleitung von einem Immunoglobulin, das nicht mit CK-MB kreuzreagiert blockiert. Nach Überleiten einer CK-MB-haltigen Lösung wird die Assoziation bis zum Erreichen des Gleichgewichts verfolgt und anschließend die zu untersuchenden potentiellen Sandwichpartner zugegeben und die Assoziation des Sandwichkomplexes bis zum Gleichgewicht verfolgt. Hierbei hat sich ergeben, daß die in der folgenden Tabelle 2 aufgeführten Kombinationen der erfindungsgemäßen Antikörper für einen Nachweis von CK-MB geeignet sind. Als besonders geeignet haben sich die Kombinationen von DSM ACC 2057 mit DSM ACC 2058 oder DSM ACC 2060 sowie die Kombinationen von DSM ACC 2059 mit DSM ACC 2058 bzw. DSM ACC 2060 erwiesen. Mit dem bisherigen Standardantikörper gegen CK-MB (ATCC HB 8939) konnte dagegen nur eine schwache Sandwichbildung beobachtet werden, die für einen zuverlässigen diagnostischen Nachweis nicht als ausreichend anzusehen ist.

**Tabelle 2**

| erster Antikörper | für einen Sandwich möglicher zweiter Antikörper |
|---|---|
| DSM ACC 2057 | DSM ACC 2058 |
| | DSM ACC 2060 |
| | |
| DSM ACC 2059 | DSM ACC 2058 |
| | DSM ACC 2060 |
| | |
| DSM ACC 2059 | DSM ACC 2057 |

Unter Verwendung der monoklonalen Antikörper DSM ACC 2057 und DSM ACC 2059 kann mit den in der folgenden Tabelle 3 angegebenen Kombinationen auch eine Tripletbildung erreicht werden, bei welcher drei Antikörper an CK-MB binden. Hierdurch kann gegenüber dem normalen Sandwich-Assay eine weitere Steigerung von Sensitivität und Spezifität erreicht werden.

**Tabelle 3**

| erster Antikörper | zweiter Antikörper | dritter Antikörper |
|---|---|---|
| DSM ACC 2057 | DSM ACC 2058 | DSM ACC 2060 |
| DSM ACC 2059 | DSM ACC 2058 | DSM ACC 2060 |

### Beispiel 4

### Bestimmung der Epitopüberlappung von Antikörpern gegen CK-MB

Zum Nachweis der Epitopüberlappung eines Antikörpers mit einem erfindungsgemäßen Antikörper wird ein kompetitiver Enzymimmunoassay durchgeführt. Dazu wird CK-MB zunächst mit D-Biotinyl-ε-Amidocapronsäure-N-hydroxysuccinimidester (Boehringer Mannheim GmbH, Kat.-Nr. 1008960) gemäß Angaben des Herstellers biotinyliert. Von diesem biotinylierten Antigen werden 300 ng in einem Volumen von 100 µl PBS durch 1-stündige Inkubation bei Raumtemperatur an eine mit Streptavidin beschichtete Mikrotiterplatte (Herstellung nach EP-A 0 344 578) gebunden. Nach 3-maligem Waschen mit PBS/ 0,05 % Tween 20 wird 90 min. bei Raumtemperatur simultan inkubiert mit dem Referenzantikörper (z.B. DSM ACC 2059), der mit Peroxidase markiert wurde (Endkonzentration 52 mU/ml), und dem zu beurteilenden Antikörper. Nach erneutem 3-maligen Waschen mit PBS/0,05 % Tween 20 wird mit der Enzymsubstratlösung ABTS® in Natriumperborat enthaltendem Puffer 30 min. bei Raumtemperatur inkubiert und anschließend die Extinktion bei 405 nm als Maß für die Menge des gebundenen POD-markierten monoklonalen Antikörpers gemessen. Dieser Wert wird verglichen mit der Extinktion, die erhalten wird bei Inkubation mit dem markierten monoklonalen Antikörper allein. Wenn bis zu einem 10⁵fachen Überschuß an zu beurteilendem Antikörper gegenüber dem markierten Antikörper mindestens 50 % Kompetition zu erkennen sind, liegt eine Epitopüberlappung vor.

Die Antikörper aus den Zellinien DSM ACC 2057 und DSM ACC 2059 zeigten eine starke Kompetition, das heißt sie erkennen beziehungsweise binden an das selbe Epitop, welches auch vom sogenannten "Conan"-Antikörper erkannt wird. Die Antikörper aus den Zellinien DSM ACC 2058, DSM ACC 2060 und DSM ACC 2168 kompetitieren nicht mit den Antikörpern der beiden anderen Zellinien (DSM ACC 2057 und DSM ACC 2059). Mit Ausnahme der beiden Antikörper aus den Zellinien DSM ACC 2060 und DSM ACC 2168 zeigen diese drei Antikörper keine Kompetition. Die Antikörper DSM ACC 2060 und DSM ACC 2168 zeigen eine geringe Kompetition, was darauf schließen läßt, daß die beiden Epitope gering überlappen. Es ist daher nicht vorteilhaft, diese beiden Antikörper beispielsweise gemeinsam in einem Sandwich-Test einzusetzen. In Kombination mit einem anderen erfindungsgemäßen Antikörper ist jeder dieser Antikörper jedoch sehr gut zu verwenden.

### Beispiel 5

### Bestimmung von CK-MB über einen LPIA-Test

Zunächst werden zwei für einen Sandwich-Test geeignete erfindungsgemäße Antikörper ausgewählt (siehe Beispiel 3) und Fab'-Fragmente gemäß den Angaben in EP-A 0 464 554 biotinyliert.

Jeweils 20 µl eines CK-MB-Standards (0, 110 und 220 ng CK-MB/ml Humanserum) bzw. die zu analysierende Probe werden an einem Hitachi 717 Photometer zum Zeitpunkt t = 0 min. als Probe einpipettiert. Hierzu werden sofort durch das Gerät automatisch 330 µl Reagenz 1 (Reaktionspuffer = 50 mmol/l Tris/HCl, pH 7,5, 75 mmol/l Natriumchlorid, 3 % PEG 35.000, 1 % Pluronic F68, 0,1 % Brij 35 und 0,1 % Natriumazid) zupipettiert. Zum Zeitpunkt t = 4,5 min. werden durch das Gerät 50 µl Reagenz 2 (Latexsuspension; 0,083 % Streptavidinlatex vorbeschichtet mit jeweils 1,6 µg Antikörper/ml in 200 mmol/l Glycin, pH 7,5, 2 % Saccharose, 0,5 % RSA I (RSA, Reinheitsstufe I, 0,1 % Natriumazid) zugegeben. Anschließend wird die Zunahme der Agglutination bichromatisch bei 340 nm (Hauptwellenlänge) und 700 nm (Nebenwellenlänge) gemessen (Meßtemperatur 37°C). Als Signalgröße wird die Extinktionsdifferenz zwischen den Zeitpunkten t = 5,3 min. und t = 9,5 min. verwendet. Hierbei hat sich die Kombination der Antikörper DSM ACC 2059 und DSM ACC 2058 als besonders geeignet erwiesen (entsprechend einer hohen Dynamik (Differenz zwischen 2. Meßwert und Leerwert) von 342 mE sowie einer hohen Anfangssteigerung der Eichkurve (2,3 mE x ml/ng).

### Beispiel 6

### Bestimmung von CK-MB über einen Sandwich-Test nach dem Elektrochemilumineszenz-Verfahren

Das ECL-Verfahren wurde entsprechend WO 86/02734, WO 87/06706 und WO 92/14138 durchgeführt. Der monoklonale Antikörper DSM ACC 2059 wurde nach Methoden des Standes der Technik biotinyliert (Lösung 2). Der monoklonale Antikörper DSM ACC 2058 wurde mit DSS (Disuccinyl-Substrat) ruthenyliert ((Tris)(2,2'-Bipyridyl)Rutheniumchloridhexahydrat)(Lösung 3). Beide Antikörper wurden 5 Minuten mit der CK-MB-haltigen Probe in Lösung 1 inkubiert und anschließend Streptavidin-beschichtete Magnetpartikel (Dynabeads M-280 Streptavidin der Firma Deutsche Dynal GmbH, Deutschland) zugesetzt (Lösung 4) und erneut 5 Minuten inkubiert.

Die Reaktion wird durch Absaugen des Reaktionsgemisches in die Meßzelle beendet. Zur Anwendung kam ein Origen 1.0-Gerät der Firma Igen, USA. In der Meßkammer werden die Magnetpartikel durch einen Magneten auf der Elektrode zurückgehaiten. Das restliche Reaktionsgemisch wird abgsaugt und die Meßkammer mit Assay-Puffer (Lösung 5) gefüllt. Die Lichtemission wird durch Anlegen einer elektrischen Spannung angeregt.

Folgende Lösungen wurden verwendet:#

### Lösung 1:

Inkubationspuffer:
100 mM Natriumphosphat pH 7,0
0,1 % RSA
0,1 % Methylisothiazolon
0,1 % Natriumbenzoat

### Lösung 2:

Biotinylierter MAK DSM ACC 2059
2 µg/ml in Inkubationspuffer

### Lösung 3:

Ruthenium-Bispyridyl markierter MAK DSM ACC 2058 2 µg/ml in Inkubationspuffer

### Lösung 4:

500 µg/ml Streptavidin-beschichtete Magnetpartikel in:
50 mM Hepes, 0,1 % RSA, 0,1 % Thesit, 0,1 % Chloracetamid, 0,01 % Methylisothiazolon, pH 7,0

### Lösung 5:

Assay Puffer: pH 6,8
Tris-Proypl-Amin 0,16 M, Di-Kaliumpydrogenphosphat 0,2 M, Polydocanol (Thesit) 0,1 %, Oxaban A 0,1 %.

## Patentansprüche

1. Verfahren zum diagnostischen Nachweis von CK-MB in einem Sandwich-Immunoassay, **dadurch gekennzeichnet dass** man mindestens zwei monoklonale Antikörper verwendet, die an das CK-MB-Isoenzym, nicht aber an die B- oder M-Untereinheit allein der CK-MB oder nicht wesentlich an die CK-MM und CK-BB Isoenzyme binden, die nicht miteinander um die selbe Bindestelle an CK-MB konkurrieren, wobei mindestens einer der monoklonalen Antikörper eine Affinitätskonstante von mindestens 1 x 10⁸ mol⁻¹ x 1 zu CK-MB aufweist.

2. Verfahren gemäß Anspruch 1, **dadurch gekennzeichnet, dass** es sich bei besagtem Sandwich-Immunoassay um einen homogenen Latex-Partikel-Immunoassay handelt.

3. Verfahren gemäß Anspruch 2, **dadurch gekennzeichnet, dass** die besagten monoklonalen Antikörper an Latex-Partikel gebunden sind und die Agglutinationszunahme als Maß für die Menge an CK-MB in der Probe bestimmt wird.

4. Verfahren gemäß Anspruch 3, **dadurch gekennzeichnet, dass** als einer der latexgebundenen Antikörper ein Antikörper verwendet wird, welcher von der Hybridomlinie DSM ACC 2058 oder DSM ACC 2060 produziert wird.

5. Verfahren gemäß Anspruch 1 **dadurch gekennzeichnet, dass** einer der besagten monoklonalen Antikörper an eine feste Phase bindet und nach Inkubation mit der zu bestimmenden Probe, einem weiteren markierten Antikörper, der an CK-MB bindet, ohne den ersten Antikörper aus seiner Bindung an CK-MB zu verdrängen, und Trennung von fester und flüssiger Phase die Markierung in einer der beiden Phasen als Maß für die Menge an CK-MB in der Probe bestimmt wird.

6. Verfahren gemäß Anspruch 5, **dadurch gekennzeichnet, dass** als einer der beiden Antikörper im Sandwich-Immunoassay ein Antikörper verwendet wird, der von der Hybridomlinie DSM ACC 2058 oder DSM ACC 2060 produziert wird.

7. Monoklonaler Antikörper gegen das CK-MB-Isoenzym, der erhältlich ist aus einer der Hybridomlinien DSM ACC 2059, DSM ACC 2057, DSM ACC 2058, DSM ACC 2060 und/oder DSM ACC 2168.

8. Hybridomzelllinie, die einen monoklonalen Antikörper gegen CK-MB produziert und ausgewählt ist aus der Gruppe DSM ACC 2059, DSM ACC 2057, DSM ACC 2058, DSM ACC 2060 und DSM ACC 2168.

9. Verwendung eines monoklonalen Antikörpers gemäß Anspruch 7 in einem Verfahren zur Bestimmung von CK-MB in einem immunologischen diagnostischen Test, gemäß einem der Ansprüche 1 - 6.

## Claims

1. Method for the diagnostic detection of CK-MB in a sandwich immunoassay, **characterized in that** at least two monoclonal antibodies are used which bind to the CK-MB isoenzyme but not to the B or M subunit alone of CK-MB or not substantially to the CK-MM and CK-BB isoenzymes, which do not compete with one another for the same binding site on CK-MB and at least one of the monoclonal antibodies having an affinity constant of at least 1 x 10⁸ mol⁻¹ x 1 for CK-MB.

2. Method as claimed in claim 1, **characterized in that** the said sandwich immunoassay is a homogeneous latex particle immunoassay.

3. Method as claimed in claim 2, **characterized in that** the said monoclonal antibodies are bound to latex particles and the increase in agglutination is determined as a measure for the amount of CK-MB in the sample.

4. Method as claimed in claim 3, **characterized in that** an antibody that is produced by the hybridoma line DSM ACC 2058 or DSM ACC 2060 is used as one of the latex-bound antibodies.

5. Method as claimed in claim 1, **characterized in that** one of the said monoclonal antibodies binds to a solid phase and, after incubation with the sample to be determined and a further labelled antibody which binds to CK-MB without displacing the first antibody from its binding to CK-MB and separating the solid and liquid phase, the label is determined in one of the two phases as a measure for the amount of CK-MB in the sample.

6. Method as claimed in claim 5, **characterized in that** an antibody is used as one of the two antibodies in a sandwich immunoassay which is produced by the hybridoma line DSM ACC 2058 or DSM ACC 2060.

7. Monoclonal antibody against the CK-MB isoenzyme which is obtainable from the hybridoma lines DSM ACC 2059, DSM ACC 2057, DSM ACC 2058, DSM ACC 2060 and/or DSM ACC 2168.

8. Hybridoma cell line which produces a monoclonal antibody against CK-MB and is selected from the group DSM ACC 2059, DSM ACC 2057, DSM ACC 2058, DSM ACC 2060 and DSM ACC 2168.

9. Use of a monoclonal antibody as claimed in claim 7 in a method for determining CK-MB in an immunological diagnostic test according to one of the claims 1 - 6.

## Revendications

1. Procédé pour le décèlement diagnostique de CK-MB dans un immunodosage en sandwich, **caractérisé en ce qu'**on utilise au moins deux anticorps monoclonaux qui se lient à l'isoenzyme CK-MB, sans toutefois se lier uniquement à la sous-unité B ou M de la CK-MB ou sans se lier de manière essentielle aux isoenzymes CK-MM et CK-BB, qui n'entrent pas en concurrence réciproque pour le même site de liaison sur la CK-MB, dans lequel au moins un des anticorps monoclonaux présente une constante d'affinité s'élevant à au moins 1 x 10⁸ mole⁻¹ x I par rapport à la CK-MB.

2. Procédé selon la revendication 1, **caractérisé en ce que**, en ce qui concerne ledit immunodosage en sandwich, il s'agit d'un immunodosage de type homogène faisant appel à des particules de latex.

3. Procédé selon la revendication 2, **caractérisé en ce que** lesdits anticorps monoclonaux se lient à des particules de latex et l'augmentation de l'agglutination est déterminée comme mesure pour la quantité de CK-MB dans l'échantillon.

4. Procédé selon la revendication 3, **caractérisé en ce qu'**on utilise, à titre d'un des anticorps liés à du latex, un anticorps qui est produit par la lignée d'hybridome DSM ACC 2058 ou DSM ACC 2060.

5. Procédé selon la revendication 1, **caractérisé en ce qu'**un des anticorps monoclonaux en question se lie à une phase solide, et après incubation avec l'échantillon à déterminer et avec un autre anticorps marqué qui se lie à CK-MB, sans repousser le premier anticorps à l'écart de sa liaison avec CK-MB, et après séparation de la phase solide et de la phase liquide, le marquage dans une des deux phases est déterminé comme mesure pour la quantité de CK-MB dans l'échantillon.

6. Procédé selon la revendication 5, **caractérisé en ce qu'**on utilise, à titre d'un des deux anticorps dans l'immunodosage en sandwich, un anticorps qui est produit par la lignée d'hybridome DSM ACC 2058 ou DSM ACC 2060.

7. Anticorps monoclonal dirigé contre l'isoenzyme CK-MB, que l'on obtient à partir d'une des lignées d'hybridomes DSM ACC 2059, DSM ACC 2057, DSM ACC 2058, DSM ACC 2060 et/ou DSM ACC 2168.

8. Lignée cellulaire d'hybridome qui produit un anticorps monoclonal dirigé contre CK-MB et qui est choisie parmi le groupe comprenant DSM ACC 2059, DSM ACC 2057, DSM ACC 2058, DSM ACC 2060 et DSM ACC 2168.

9. Utilisation d'un anticorps monoclonal selon la revendication 7, dans un procédé pour la détermination de CK-MB dans un essai diagnostique immunologique, selon l'une quelconque des revendications 1 à 6.
